# EUROPEAN PATENT APPLICATION

(11) **EP 4 306 505 A1**
(43) Date of publication of application: **17.01.2024**
(21) Application number: 22767139.3
(22) Date of filing: 08.03.2022
(51) Int. Cl.: C07C 17/354, C07B 61/00, C07C 17/25, C07C 19/08, C07C 21/18

(54) **METHOD FOR PREPARING 1,1,2-TRIFLUOROETHANE**

(30) Priority: 09.03.2021 JP 2021036855
(71) Applicant: DAIKIN INDUSTRIES, LTD., Osaka-Shi, Osaka 530-0001 (JP)
(72) Inventor: TAKAHASHI, Kazuhiro, Osaka-Shi, Osaka 530-0001 (JP); CHAKI, Takehiro, Osaka-Shi, Osaka 530-0001 (JP); NAKAUE, Tsubasa, Osaka-Shi, Osaka 530-0001 (JP); IWAMOTO, Tomoyuki, Osaka-Shi, Osaka 530-0001 (JP); KUSHIDA, Megumi, Osaka-Shi, Osaka 530-0001 (JP)
(74) Representative: Hoffmann Eitle
(86) International application number: PCT/JP2022/010001
(87) International publication number: WO 2022/191185

(57) **Abstract**

An object is to provide an efficient method for producing HFC-143 or HFO-1132. Provided as a means for solution is a method for producing 1,1,2-trifluoroethane (HFC-143), comprising subjecting 1,1,2-trifluoroethylene to a reduction reaction with a reducing agent in the presence of a catalyst to obtain HFC-143.

## Description

### Technical Field

The present disclosure relates to methods for producing 1,1,2-trifluoroethane (HFC-143) and 1,2-difluoroethylene (HFO-1132) .

### Background Art

Instead of CFCs, HCFCs, or the like that destroy the ozone layer, HFC-134a (C₂H₂F₄), HFC-125 (C₂HF₅), HFC-32 (CH₂F₂), and R410A, which is a mixed refrigerant of HFC-32 and HFC-125, have been used.

However, the global warming potential (GWP) of R410A is 2088, and various low-GWP refrigerants that can be alternatives for R410A are being investigated due to growing concerns about global warming.

As a method for producing HFO-1132(E), which is the trans isomer of 1,2-difluoroethylene (hereinafter also simply referred to as "HFO-1132"), which is a type of such alternative refrigerant, PTL 1 discloses a method for thermally decomposing CHFX (wherein X is Cl or F) .

Further, PTL 2 discloses a method for obtaining HFO-1132 by using haloethane or haloethylene to produce HFC-142 or HFC-143, followed by dehydrofluorination.

### Citation List

### Patent Literature

PTL 1: JP2013-241348A
PTL 2: JP2019-196312A

### Summary of Invention

### Technical Problem

In view of the above circumstances, an object of the present disclosure is to provide an efficient method for producing HFC-143 or HFO-1132.

### Solution to Problem

The present disclosure includes the following configurations.

### Item 1.

A method for producing 1,1,2-trifluoroethane (HFC-143), comprising subjecting 1,1,2-trifluoroethylene to a reduction reaction with a reducing agent to obtain HFC-143.

### Item 2.

The production method according to Item 1, wherein the reduction reaction is performed at 100 to 500°C.

### Item 3.

The production method according to Item 1 or 2, wherein the reducing agent contains hydrogen.

### Item 4.

A method for producing 1,2-difluoroethylene (HFO-1132), comprising subjecting HFC-143 obtained by the production method according to any one of Items 1 to 3 to dehydrofluorination to obtain HFO-1132.

### Advantageous Effects of Invention

According to the method for producing HFC-143 of the present disclosure, HFC-143 can be efficiently obtained. Further, HFO-1132 can be efficiently obtained by using this production method.

### Brief Description of Drawing

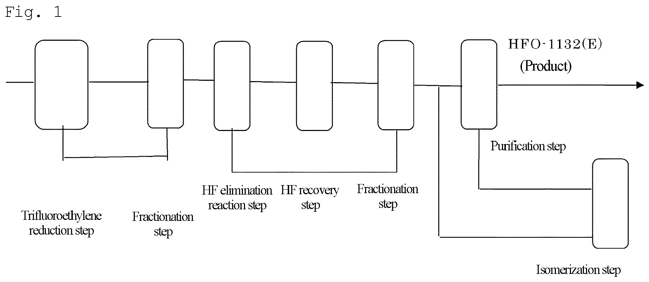

Fig. 1 shows an example of the production process of HFC-143 or HFO-1132 according to the present disclosure.

### Description of Embodiments

### Method for Producing HFC-143

The method for producing HFC-143 of the present disclosure comprises subjecting 1,1,2-trifluoroethylene to a reduction reaction with a reducing agent to obtain HFC-143.

1,1,2-Trifluoroethylene can be obtained by a known method. For example, HFO-1123 can be obtained based on the method disclosed in US2802887A. Specifically, it is indicated that 1,1,2-trifluoroethylene is obtained at a high yield when using chlorotrifluoroethylene (hereinafter referred to as "CTFE") as a raw material and a 5% catalyst supported on activated carbon as a catalyst at a reaction temperature of 100°C to 160°C, a contact time of 7 to 15 seconds, and a hydrogen/CTFE ratio of 0.5 to 1.0.

The reducing agent used may be an inorganic or organic reducing agent. Specific examples include hydrogen, formic acid, ammonium formate, sodium formate, formic acid-triethylamine, triethylsilane, tetramethyldisiloxane, polymethylhydrosiloxane, NaBH₃CN, NHCBH₃ (N-heterocyclic carbene boranes), and nitrogen-containing unsaturated heterocyclic compounds having an N-H moiety (imino group). Of these, as the reducing agent used when a gas-phase reduction reaction is performed in the presence of a catalyst, hydrogen, hydrogen sulfide, carbon monoxide, methane, or the like can be used, and hydrogen is particularly preferably used.

The amount of hydrogen when used as a reducing agent is preferably 0.5 to 20 mol, and more preferably 1.0 to 10 mol, per mol of 1,1,2-trifluoroethylene. When another reducing agent is used, the amount of reducing agent is preferably 0.5 to 30 mol, and more preferably 1.0 to 20 mol, per mol of 1,1,2-trifluoroethylene. By adopting such a configuration, the required amount of reducing agent is contained, whereby deterioration of the catalyst can be suppressed, and the target product HFC-143 can be obtained with a high selectivity. Further, because an excessive amount of reducing agent is not contained, product losses associated with the recovery of unreacted hydrogen can be reduced.

In the method for producing HFC-143 of the present disclosure, a catalyst is preferably used. As the catalyst, a wide range of known reducing catalysts can be used, and there is no limitation thereto. Specific examples include catalysts supported on metals such as Pd, Pt, Rh, Ru, or Re, activated carbon, metal oxides such as alumina, or metal fluorides. As a reduction reaction mechanism using a catalyst, a mechanism in which hydrogen atoms are activated on the metal surface and reacted with organic substances is known. Therefore, metals having such effects can be widely used. Among them, it is preferable to use Pd or Pt. The metal loading ratio when using a supported catalyst is preferably 0.1 to 20 mass%, more preferably 0.1 to 10 mass%, and even more preferably 0.5 to 5 mass%.

According to the method for producing HFC-143 of the present disclosure, the deterioration of the catalyst is extremely low. As a result, compared with the invention disclosed in PTL 2 mentioned above, it is possible to reduce the production cost by procuring raw materials, and reduce the size of the equipment by eliminating the need to regenerate the deteriorated catalyst.

The method for producing HFC-143 of the present disclosure is preferably performed in the gas phase in the presence of a catalyst. This is because the gas-phase reaction has advantages over the liquid-phase reaction in terms of the recovery of products and unreacted raw materials, and the separation of the catalyst and products.

The reaction temperature is preferably set within the range of 100 to 500°C, more preferably 120 to 400°C, and even more preferably 150 to 300°C.

The pressure during reaction is not limited, and the reaction can proceed under reduced pressure, normal pressure, or increased pressure. Above all, for the reasons of reducing the loss of raw materials, such as hydrogen and 1,1,2-trifluoroethylene, and the product 1,1,2-trifluoroethane in the subsequent separation step, and reducing the operating energy, it is preferable to perform the reaction at a pressure in the range of 0.1 to 2 MPaG.

Regarding the reaction time, W/F0, wherein W is the weight of catalyst and F0 is the flow rate of raw material (F0 is the flow rate at 0°C and 0.1013 MPa), is preferably 0.1 to 60, and more preferably 1 to 50.

### Method for Producing HFO-1132

The present disclosure includes an invention relating to a method for producing HFO-1132, comprising subjecting HFC-143 obtained by the method for producing HFC-143 described above to dehydrofluorination, thereby obtaining HFO-1132. This invention can be, for example, as shown in the following reaction formula 1, a method for producing HFO-1132, comprising step (a) of reacting 1,1,2-trifluoroethylene with a reducing agent to obtain HFC-143, and step (b) of subjecting HFC-143 obtained in step (a) to dehydrofluorination to obtain HFO-1132.

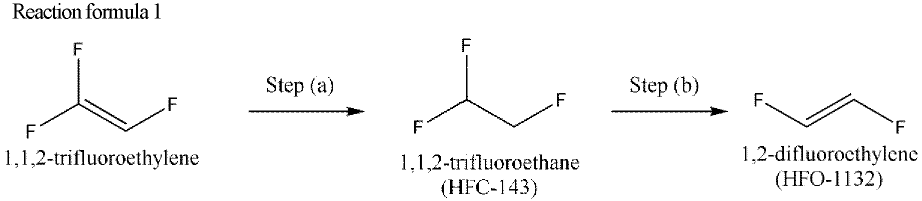

### Step (a)

In step (a), 1,1,2-trifluoroethylene and a reducing agent are reacted in the presence of a catalyst to obtain HFC-143. Step (a) can be performed based on the method for producing HFC-143 described above.

### Step (b)

In step (b), HFC-143 obtained in step (a) is subjected to dehydrofluorination to obtain HFO-1132.

In step (b), it is preferable to use a dehydrofluorination catalyst. As the dehydrofluorination catalyst, any known catalyst active for the fluorination reaction with hydrogen fluoride can be used. Usable examples include metal oxides and fluorinated metal oxides, such as chromium oxide, chromium oxide fluoride, aluminum fluoride, and aluminum oxide fluoride. Other usable examples include metal fluorides, such as MgF₂, TaF₅, and SbF₅.

Among these catalysts, for example, chromium oxide is not limited; however, it is preferable to use one represented by the composition formula: CrOₘ, wherein m is 1.5<m<3. The chromium oxide catalyst may be in powder form, pellet form, or any form suitable for the reaction, with the pellet form being preferred.

Oxygen is preferably supplied together with HFC-143 to the reactor. The amount of oxygen supplied is preferably 0.5 to 20 mol, and more preferably 1 to 10 mol, per mol of HFC-143. Within this numerical range, it is possible to obtain the effect of suppressing the deterioration of the catalyst and stably performing the reaction.

The reaction temperature in step (b) is preferably, as the temperature in the reactor, 300 to 500°C, and more preferably 350 to 450°C. By setting the reaction temperature to these sufficiently high temperatures, a high yield can be ensured. Further, by keeping the reaction temperature below these excessive temperatures, it is possible to suppress the formation of impurities and prevent irreversible catalyst deterioration due to structural changes in the catalyst.

The pressure during reaction is preferably set so that HFC-143 can exist in a vapor state, and the pressure may be normal pressure or increased pressure. Specifically, the reaction is preferably performed at a pressure of 0 to 1 MPaG, and more preferably 0.05 MPaG to 0.7 MPaG.

The reaction time is not limited, and the contact time represented by the ratio of the catalyst loading W (g) to the flow rate Fo of raw material gas flowing into the reaction system (flow rate at 0°C and an atmospheric pressure of 0.1013 MPa: cc/sec): W/Fo, may be set within the range of about 10 to 100 g·sec/cc, and preferably about 20 to 60 g-sec/cc.

HFO-1132 obtained through step (b) contains both cis-HFO-1132(Z) and trans-HFO-1132(E). By appropriately setting the reaction conditions, it is possible to obtain HFO-1132(E) with a high selectivity of 1 to 30 mass%, and preferably 5 to 20 mass%, in the total amount of HFO-1132.

In the method disclosed in PTL 1, there is concern about energy loss due to the high-temperature reaction characteristic of pyrolysis reactions, and excessive by-products are produced. In addition, the yield of HFO-1132(E) is extremely low. According to the method for producing HFO-1132 of the present disclosure, HFO-1132(E) can be obtained at a high yield.

### Step (c)

HFO-1132 obtained in step (b) contains HFO-1132(E) and HFO-1132(Z). In order to obtain only HFO-1132(E) from the mixture containing HFO-1132(E) and HFO-1132(Z), or increase the content of HFO-1132(E), it is also preferable to provide step (c) as an isomerization step after step (b).

As the catalyst used in the isomerization reaction in step (c), a chromium-containing catalyst is preferably used, and chromium oxide is particularly preferably used. Examples of chromium oxide include crystalline chromium oxide, amorphous chromium oxide, and the like.

The chromium oxide is not limited, and is preferably, for example, chromium oxide represented by the composition formula: CrOₘ, wherein 1.5<m<3, more preferably 2<m<2.75, and even more preferably 2<m<2.3. The chromium oxide catalyst can be used in any form, such as powder or pellets, as long as it is suitable for the reaction. It is also particularly preferable to use a fluorinated chromium oxide catalyst. The chromium oxide is preferably pretreated with anhydrous hydrogen fluoride. As a pretreatment method of chromium oxide as a precursor with anhydrous hydrogen fluoride, for example, the method disclosed in JPH05-146680A (corresponding to US6337299B1) can be used.

The reaction temperature is not limited and can be appropriately set. Specifically, the reaction temperature is preferably set within the range of about 180°C to 500°C, and more preferably within the range of about 200°C to 400°C.

The reaction time is not limited and can be appropriately set. Although the conversion can be increased by lengthening the contact time, the amount of catalyst used increases, requiring larger facilities, which leads to inefficiency. In general, the contact time represented by the ratio of the catalyst loading W (g) to the flow rate Fo of raw material gas flowing into the reaction system (flow rate at 0°C and 1 atm: cc/sec): W/Fo, may be set within the range of about 10 to 80 g·sec/cc, and preferably within the range of about 20 to 60 g·sec/cc.

The pressure of the reactor is not limited and can be appropriately set. Since a high pressure promotes the formation of polymers, such as tar, an appropriate pressure can be set. In general, the pressure may be set within the range of about 0 to 1 MPaG, and preferably within the range of about 0 to 0.5 MPaG.

Next, a more specific example of the method for producing HFC-143 or HFO-1132 of the present disclosure is described based on Fig. 1.

As described above, in step (a), 1,1,2-trifluoroethylene is reacted with a reducing agent to obtain HFC-143. The target product in this step is HFC-143, and intermediates other than this product are recycled to the reactor.

In step (b), HFC-143 is subjected to dehydrohalogenation to obtain HFO-1132. Usable catalysts include aluminum oxide, chromium oxide, and aluminum oxide fluoride and chromium oxide fluoride catalysts obtained by the fluorination of these oxides. The chromium oxide fluoride catalyst can be prepared by the method described above.

In step (b), 1,2-difluoroethylene (Z), which is a cis-trans isomer, is produced in addition to trans-1,2-difluoroethylene. In step (c), the composition containing both isomers can be subjected to an isomerization reaction to thereby obtain 1,2-difluoroethylene (E).

The isomerization reaction can be performed in the presence or absence of a catalyst. This reaction can also be performed by photoreaction by irradiation with light having a wavelength in the region from ultraviolet to vacuum-ultraviolet, and depending on the case, it can be performed in coexistence with a photosensitizer. These reactions can be performed in either the gas or liquid phase.

In each step, of the intermediates that have not been converted to HFC-143 or HFO-1132, components that can be used as reaction raw materials are preferably separated in the subsequent distillation step and then recycled to the reaction step.

The embodiments of the present disclosure are described above; however, the present disclosure is not limited to these examples. Of course, the present disclosure can be embodied in various forms without departing from the gist of the present disclosure.

### Examples

Embodiments of the present disclosure are described in more detail below based on Examples; however, the present disclosure is not limited thereto.

### Example 1

### Production of HFC-143 from 1,1,2-Trifluoroethylene/Step (a)

1 kg of a 1.5 mass% Pd-supported activated carbon catalyst was packed in a SUS304 reaction tube with an inner diameter of 50 mm, and dried at 200°C for 3 hours under a nitrogen purge. Then, 1,1,2-trifluoroethylene at 300 Nml/min and hydrogen at 1000 Nml/min ("Nml/min" indicates the gas flow rate at 0°C and an atmospheric pressure of 0.1013 MPa) were supplied, and a reduction reaction was performed at a reaction temperature of 200°C, a contact time (W/F0) of 20, and a reaction pressure of 0.1 MPaG. The outlet gas was washed with water, dried with calcium chloride, and then analyzed by GC (Shimadzu GC-2030) equipped with a GS-GASPRO capillary column (0.32 mm × 60 m). Table 1 below shows the conversion of 1,1,2-trifluoroethylene and the selectivity of HFC-143 at each reaction temperature.

**Table 1**

| Reaction temperature (°C) | 1,1,2-Trifluoroethylene conversion | HFC-143 selectivity |
|---|---|---|
| 150 | 56 | 100 |
| 200 | 70 | 100 |
| 250 | 82 | 97 |
| 300 | 93 | 93 |

### Example 2

### Production of HFO-1132 from HFC-143/Step (b)

As a fluorination catalyst, 10 g (fluorine content: about 15.0 mass%) of a catalyst obtained by the fluorination of chromium oxide represented by the composition formula: CrO₂ was packed in a tubular Hastelloy reactor with an inner diameter of 15 mm and a length of 1 m. The reaction tube was maintained at 400°C under atmospheric pressure (0.1013 MPa), and HFC-143 was supplied at a flow rate of 15.0 Nml/min. The contact time W/Fo was 40.0 g·sec/Nml. 4 hours after the start of the reaction, the conversion of HFC-143 was 98%, and the selectivity of HFO-1132 was 89%. The molar ratio of two isomers of HFO-1132, i.e., 1,2-difluoroethylene (E) and 1,2-difluoroethylene (Z), (1,2-difluoroethylene (E)/1,2-difluoroethylene (Z)) was 19/81.

### Example 3

### Isomerization Reaction/Step (c)

10 g of a catalyst obtained by the fluorination of chromium oxide represented by CrO₂, similar to the one used in Example 2, was packed in a reactor with an outer diameter of 12.7 mm and a length of 700 mm, and an isomerization reaction was performed. A raw material gas obtained by adding nitrogen and oxygen to 1,2-difluoroethylene (Z) to prevent the deterioration of the catalyst was supplied to the reactor at a rate of 6 to 10 Nml/min, and an isomerization reaction was performed at a reaction temperature of 250 to 300°C. Table 1 shows the reaction results.

**Table 2**

| cis-1132 | N2 | 02 | Temperature | W/F0 | Flow rate | Organic substance outlet composition (mol%) | | | | |
|---|---|---|---|---|---|---|---|---|---|---|
| | | | | | | 1132(E) | 1132(Z) | 143a | 134a | Other |
| (mol%) | (mol%) | (mol%) | (°C) | (g/Nml·s) | (Nml/s) | | | | | |
| 100 | 0 | 0 | 300 | 60 | 10 | 15 | 82 | 0.2 | 1.1 | 1.7 |
| 50 | 50 | 0 | 300 | 60 | 10 | 30 | 63 | 3.5 | 1.3 | 2.2 |
| 50 | 50 | 0 | 250 | 60 | 10 | 22 | 75 | 0.4 | 1.2 | 1.4 |
| 50 | 50 | 0 | 300 | 100 | 6 | 28 | 59 | 8.4 | 1.3 | 3.3 |
| 80 | 20 | 0 | 300 | 60 | 10 | 30 | 62 | 4.3 | 1.0 | 2.7 |
| 80 | 16 | 4 | 300 | 60 | 10 | 29 | 63 | 4.1 | 1.1 | 2.8 |

## Claims

1. A method for producing 1,1,2-trifluoroethane (HFC-143), comprising subjecting 1,1,2-trifluoroethylene to a reduction reaction with a reducing agent to obtain HFC-143.

2. The production method according to claim 1, wherein the reduction reaction is performed at 100 to 500°C.

3. The production method according to claim 1 or 2,
wherein the reducing agent contains hydrogen.

4. A method for producing 1,2-difluoroethylene (HFO-1132), comprising subjecting HFC-143 obtained by the production method according to any one of claims 1 to 3 to dehydrofluorination to obtain HFO-1132.
